# EUROPEAN PATENT APPLICATION

(11) **EP 3 858 968 A1**
(43) Date of publication of application: **04.08.2021**
(21) Application number: 20155113.2
(22) Date of filing: 03.02.2020
(51) Int. Cl.: C12M 1/00

(54) **PRETREATMENT ARRANGEMENT COMPRISING A SLUICE VESSEL**

(71) Applicant: Sekab E-Technology AB, 891 26 Örnsköldsvik (SE)
(72) Inventor: SJÖBLOM, Anders, 891 78 BONÄSSUND (SE); CAVKA, Adnan, 891 96 ARNÄSVALL (SE); SUNDVALL, Elias, 892 32 DOMSJÖ (SE)
(74) Representative: Kransell & Wennborg KB

(57) **Abstract**

The present disclosure generally relates to a pretreatment arrangement (100) for pretreatment of lignocellulosic biomass. The pretreatment arrangement (100) comprises a reactor vessel (101) having an upstream inlet (102) for receiving biomass and a downstream outlet for discharging biomass (103). The pretreatment arrangement (100) further comprises a sluice vessel (104). The present disclosure also relates to a method (200) for pretreating lignocellulosic biomass.

## Description

### TECHNICAL FIELD

The present disclosure generally relates to a pretreatment arrangement for pretreatment of lignocellulosic biomass and to a system comprising the pretreatment arrangement. The pretreatment arrangement comprises a reactor vessel having an upstream inlet for receiving biomass and a downstream outlet for discharging biomass. The pretreatment arrangement further comprises a sluice vessel. The present disclosure also relates to a method for pretreating lignocellulosic biomass.

### BACKGROUND

Production of renewable chemicals and fuels has attained increasing interest during the last years due to environmental concerns as well as the importance of energy security. The production typically takes place in a biorefinery, and the use of lignocellulosic biomass as feedstock is an attractive route due to its abundancy and low cost.

Lignocellulosic materials are composed of cellulose, hemicellulose and lignin. Cellulose and hemicellulose can be hydrolyzed to fermentable sugars. From these sugars, various fermentation products, such as ethanol, can be produced by fermentation with microorganisms, e.g. *Saccharomyces cerevisiae.*

To facilitate the hydrolysis and subsequent fermentation to increase the yield of ethanol, a pretreatment process is typically conducted, wherein the hemicellulose is degraded, and cellulose is made more accessible as well as, to some extent, converted into fermentable sugars.

Pretreatment is an important step in the process of converting biomass into fermentation products since it has a direct effect on downstream processes and ultimate sugar yield. A typical process for pretreatment involves breaking the recalcitrant structure of the lignocellulosic biomass to increase the accessible surface of the lignocellulosic material for subsequent enzymatic hydrolysis.

For example, the recalcitrant structure may be broken in a process involving steam explosion. In such a process, the lignocellulosic biomass is steam-heated at an increased pressure during a certain time followed by a rapid discharge into atmospheric pressure, causing the biomass to explode due to the pressure drop. The release of pressure causes the biomass to disintegrate into smaller particles. Smaller particles are beneficial to increase the enzyme accessibility for subsequent hydrolysis.

The pretreatment process is typically carried out in a pretreatment arrangement, such as a pretreatment reactor. The pretreatment reactor generally comprises an inlet for receiving the biomass to be pretreated and an outlet for discharging the pretreated biomass, and a closed vessel wherein the pretreatment process is carried out.

If the pretreatment involves steam explosion, such a process poses demands on the equipment utilized. The high temperatures and pressures used within the reactor may result in the formation of deposits within the reactor, and such deposits may build up on the reactor walls. The formation of deposits may be a result of charring of the biomass and degradation of sugar and lignin. Furthermore, when the biomass is discharged from the reactor outlet, the significant pressure drop may cause undesirable fluctuations in temperature and pressure within the reactor. This may further enhance the formation of deposits within the reactor.

There is therefore a need for improvements with respect to preventing the formation of deposits during pretreatment and for overcoming problems with temperature and pressure fluctuations within the pretreatment reactor. Particularly, there is a need to provide a pretreatment system, wherein the discharge of biomass is improved and controlled without yielding significant pressure or temperature drops in the reactor.

### SUMMARY

In view of the above, it is an object of the present disclosure to provide improvements with respect to systems for pretreatment of lignocellulosic biomass, particularly with respect to reducing the formation of deposits within the reactor during operation, and to improve and control the discharge of biomass from the reactor.

According to a first aspect of the present disclosure, there is provided a pretreatment arrangement for pretreatment of lignocellulosic biomass comprising
a) a reactor vessel having an upstream inlet for receiving biomass and a downstream outlet for discharging biomass,
b) at least one sluice vessel arranged downstream of and in fluid communication with the outlet, wherein the sluice vessel comprises a first discharge valve, a second discharge valve arranged downstream of the first discharge valve and a compartment arranged between the first and the second discharge valve; the first and the second discharge valves being configured to be operable between an open and a closed position, and
c) means for increasing the pressure in the compartment of the sluice vessel.

The present inventive concept is based on the realization that the biomass can be treated in the reaction vessel under optimum conditions for pretreatment (i.e. suitable time, pressure and temperature), and the discharge of biomass is performed separate from the reactor vessel, yielding a more controlled and improved discharge of biomass. Increasing the pressure in the compartment of the sluice vessel, and subsequently releasing the biomass into atmospheric pressure upon discharge of the biomass causes the biomass to be disintegrated into smaller particles. Increasing pressure difference between discharge pressure and atmospheric pressure results in decreased particle size due to stronger disintegration of the biomass into particles. Increasing pressure requires increasing temperature, and high temperatures may cause burning or charring of sugars and biomass within the reactor vessel. As a result, deposits may build up within the reactor. Increasing the pressure in a sluice vessel arranged downstream of the pretreatment reactor allows for pretreatment processes involving steam explosion to achieve process conditions that prevent charring of biomass, degradation of sugars, and deposits to build up in the pretreatment reactor. The arrangement of the sluice vessel downstream of the pretreatment reactor allows for an increase in pressure to be achieved in the sluice vessel, thereby yielding a higher pressure difference than if the material been released directly from the pretreatment reactor. The higher pressure difference results in smaller particle size of biomass which is beneficial for subsequent hydrolysis and release of sugars from the biomass during hydrolysis, while not risking destroying sugars and biomass due to overly high temperatures and pressures under extended residence times in the pretreatment reactor.

The sluice vessel may be attached to the reactor vessel or it may be connected to the reactor vessel by means of a pipe.

In embodiments, the compartment may comprise a tank and/or a pipe
A tank may be advantageous as it allows more material to be treated or pressurized at the same time. A pipe may be advantageous as it facilitates achieving an increased pressure in a shorter time (due to the smaller size of the pipe).

In embodiments, the means for increasing the pressure is a means for supplying gas, such as steam, to the compartment. A rapid increase in pressure can thereby be achieved.

In embodiments, the sluice vessel comprises means for measuring the pressure in the sluice vessel. This is to secure that the pressure within the compartment and the sluice vessel is sufficient to enable disintegration into smaller particles and to avoid increasing the pressure to an unnecessarily extent.

In embodiments, the second discharge valve is configured to be opened in one step or in multiple steps.

In other words, the pressure drop from the increased pressure within the compartment to a lower pressure, e.g. atmospheric pressure may be performed simultaneously with discharge of the biomass. Alternatively, if the second discharge valve is configured to be opened in multiple steps, the biomass may be gradually discharged from the sluice vessel.

In embodiments, the second discharge valve is configured to be opened in multiple steps, such as two steps, wherein the first step is conducted at a lower speed than the following step(s).

This allows for a gentler, yet controlled, discharge of biomass since the time period for decreasing the pressure from the increased compartment pressure to a lower pressure (typically atmospheric pressure) is longer.

In embodiments, the reactor vessel is a vertical reactor vessel. In a vertical reactor vessel, the biomass flows from the inlet to the outlet by means of gravity and no additional means to increase the flow of biomass within the reactor vessel is required.

Preferably, the sluice vessel is adapted for steam explosion.

As mentioned hereinbefore, if steam explosion is performed upon a direct discharge of biomass from the outlet of the reactor vessel, this may cause imbalanced and impaired reaction conditions within the reactor rendering the pretreatment unstable and increasing the risk of deposit formations. A sluice vessel adapted for steam explosion reduces such risks and renders the pretreatment arrangement, particularly the discharge means, stable and controlled.

In embodiments, the sluice vessel is a first sluice vessel and wherein the pretreatment arrangement further comprises a second sluice vessel; the second sluice vessel being arranged in parallel with the first sluice vessel or downstream of the first sluice vessel.

Under certain circumstances, it may be beneficial to include at least one more sluice vessel in the pretreatment arrangement. For example, if the second sluice vessel is arranged downstream of the first sluice vessel, the pressure may increase and decrease in various steps. Steam explosion may be performed at least twice in such a set-up, wherein the first steam explosion step may result in the disintegration into particles of a larger size than the second steam explosion step, where the particles are typically smaller. If the sluice vessels are arranged in parallel, more material may be pretreated simultaneously.

In embodiments, the reactor vessel further comprises a scraping device configured to scrape deposits formed on the interior walls of said reactor vessel.

A scraping device may be arranged in the reactor vessel to prevent the formation of deposits on the interior reactor walls and to scrape off deposits potentially formed.

To further secure optimal and stable reaction conditions and to prevent undesirable temperature and pressure fluctuations within the reactor vessel, the pretreatment arrangement may comprise a gas valve configured to remove gas from the reactor vessel.

During pretreatment; i.e. during degradation or partial degradation of the biomass, gases and volatile compounds may be liberated from the biomass, resulting in the accumulation of gases in the reactor. The accumulation of gases may result in undesirable temperature and pressure fluctuations within the reactor, and eventually lead to problems with deposits in the reactor. Thus, the removal of gases from the reactor vessel during the pretreatment reaction provides for improvements with respect to maintaining balanced temperature and pressure conditions in the reactor during the pretreatment, and thereby also reducing the formation of deposits on the interior walls of the reactor.

According to a second aspect of the present disclosure, there is provided a method for pretreatment of lignocellulosic biomass comprising:
a) pretreating the lignocellulosic biomass in a pretreatment arrangement at a first pressure (p₁), wherein the pretreatment arrangement comprises a reactor vessel having an upstream inlet for receiving biomass and a downstream outlet for discharging biomass; the pretreatment arrangement further comprising a sluice vessel comprising a first discharge valve, a second discharge valve arranged downstream of the first discharge valve, and a compartment arranged between the first and the second discharge valves,
b) discharging the biomass into the compartment by opening the first discharge valve,
c) closing the first discharge valve,
d) increasing the pressure in the compartment to a second pressure (p₂),
e) discharging the biomass by opening the second discharge valve.

By increasing the pressure inside the sluice vessel to pressure (p2), a higher pressure drop when discharging the biomass from the second valve can be obtained. Consequently, the treated biomass will be divided into smaller pieces compared to if a direct discharge from the reactor would have been performed. Furthermore, the time that the biomass is kept at the increased pressure (p2), (and consequently increased temperature), compared with the time that it is exposed to the reactor pressure (p1) may be shorter.

As mentioned hereinbefore, it is advantageous to avoid significant pressure increases within the reactor vessel since this may result in burning and charring of sugars, and biomass yielding deposits.

The pressure may be increased by supplying gas, such as steam to the compartment

In embodiments, the second pressure, p₂, is 1-40 bar, such as 2-30 bar, such as 4-20 bar higher than the first pressure, p₁.

According to another aspect, there is provided a system for treatment of lignocellulosic biomass comprising a pretreatment arrangement as described hereinbefore and a hydrolysis unit arranged in fluid communication with and downstream of the pretreatment arrangement, and optionally, a fermentation unit arranged in fluid communication with and downstream of the hydrolysis unit.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Figure 1a schematically illustrates a pretreatment arrangement comprising a sluice vessel according to an exemplary embodiment of the present disclosure.
Figure 1b schematically illustrates a sluice vessel comprising a tank and a tube, which may be used in a pretreatment arrangement of the present disclosure.
Figure 2 schematically illustrates a method for pretreatment of lignocellulosic biomass according to the present disclosure.
Figure 3 schematically illustrates a system for treatment of lignocellulosic biomass according to the present disclosure.

### DETAILED DESCRIPTION

The present invention will now be described more fully hereinafter with reference to the accompanying drawings. The present invention may, however, be embodied in many different forms and should not be construed as limited to the embodiments set forth herein; rather, these embodiments are provided for thoroughness and completeness, and fully convey the scope of the present invention to the skilled person.

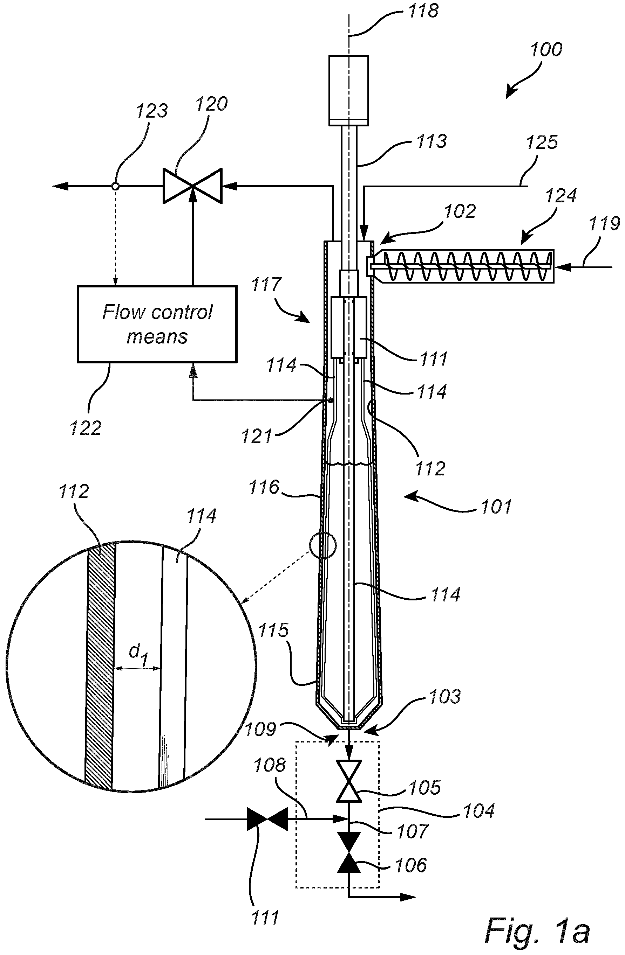

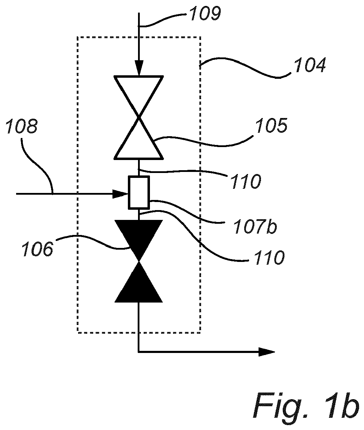

Figure 1 illustrates a pretreatment arrangement 100 for pretreatment of lignocellulosic biomass comprising
a) a reactor vessel 101 having an upstream inlet 102 for receiving biomass and a downstream outlet 103 for discharging biomass,
b) a sluice vessel 104 arranged downstream of and in fluid communication with the outlet 103, wherein the sluice vessel 104 comprises a first discharge valve 105, a second discharge valve 106 arranged downstream of the first discharge valve 105 and a compartment 107 arranged between the first 105 and the second 106 discharge valve; the first 105 and the second 106 discharge valves being configured to be operable between an open and a closed position, and
c) means 108 for increasing the pressure in the compartment 107 of the sluice vessel 104.

Lignocellulosic biomass 119 enters the reactor vessel 101 by means of the inlet 102. In figure 1, the biomass is fed into the reactor vessel 101 by means of a plug screw feeder 124. The plug screw feeder 124 secures an even flow of biomass into the reactor vessel 101 without disrupting the pressure inside the reactor vessel. The pretreatment arrangement is not limited to a specific type of inlet or means for feeding biomass, but any inlet or feeding means known to those skilled in the art may be used. The inlet and/or outlet may e.g. be a blow line or a blow valve.

The lignocellulosic biomass may be, but is not limited to hardwoods, softwoods, sugarcane bagasse, energy cane, corn stover, corn cobs, corn fibers, straw from rice, wheat, rye and other crops residues.

When the biomass slurry has been pretreated within the reactor vessel 101, the biomass is discharged from the reactor vessel 101 through the outlet 103 and enters the sluice vessel 104. The biomass is discharged by first opening the first discharge valve 105, allowing an amount of pretreated biomass to enter the compartment 107. The second discharge valve 106 is kept closed during discharge from the first discharge valve 105 into the compartment 107. The first discharge valve 105 is thereafter closed. Both discharge valves are in a closed position when the pressure is increased within the compartment 107. When the second discharge valve 106 is opened, the biomass is discharged from the compartment 107 of the sluice vessel 104. Upon discharge, the pressure drop (from the pressurized atmosphere in the compartment to the lower pressure, e.g. atmospheric pressure) causes the biomass to become disintegrated into smaller particles. Typically, this is achieved by means of steam explosion. In other words, the pretreatment arrangement 100 of the present disclosure provides for an improved and more controlled discharge of biomass. Particularly, it allows for an improved steam explosion to be carried out.

The sluice vessel 104 is arranged downstream of the reactor vessel 101. The sluice vessel 104 may be a separate component from the reactor vessel 101 or it may be an integrated component of the pretreatment arrangement 100.

In other words, the sluice vessel 104 may be attached to the reactor vessel 101 or it may be connected to the reactor vessel 101 by means of a passage, such as a pipe, a tube or a screw. In figure 1, the sluice vessel 104 is connected to the reactor vessel 101 by means of a pipe 109.

In embodiments, the compartment 107 comprises a pipe. A compartment in the form of a pipe facilitates an increase of pressure in the sluice vessel 104.

In alternative embodiments, the compartment 107 comprises a tank 107b, as illustrated in figure 1b. In figure 1b, the tank 107b is connected to the first 105 and second 106 discharge valves by means of a pipe 110. Alternatively, the first 105 and the second 106 discharge valves are directly attached to the tank 107b. For example, the discharge valves 105 and 106 may form the inlet, and the outlet, respectively of the tank.

The means 108 for increasing the pressure in the sluice vessel 104 may be connected to the tank 107b or to the pipe 110. It may be advantageous that the compartment comprises a tank since more material can be treated at the same time at an increased pressure.

As used herein, the term "tank" means a receptacle or a chamber for holding, storing and transporting biomass from the first discharge valve 105 to the second discharge valve 106. The volume of a tank is considerably larger than that of a pipe.

As indicated by the arrow 108 in figure 1, the means 108 for increasing the pressure is a means for supplying gas, such as steam, to the compartment 107.

The means to supply gas may e.g. comprise a pipe connected to the compartment 107 of the sluice vessel 104, and which pipe is connected to a suitable gas source. The means 108 is adapted to supply gas, such as steam, e.g. pressurized steam, to the compartment 107. The supply of gas into the compartment 107 causes the pressure to increase within the compartment 107.

The pressure of the gas or the steam supplied into the compartment 107 may be in the range of from 25-55 bar, such as 35-45 bar.

The means 108 for increasing pressure may further comprise a valve 111 to control the supply of gas into the compartment 107.

The sluice vessel 104 may comprise means for measuring the pressure of the sluice vessel.

The means for measuring the pressure may e.g. comprise a pressure meter, a pressure sensor or a pressure gauge. The means for controlling the pressure may be arranged in the first discharge valve 105, in the second discharge valve 106 or in the compartment 107. Preferably, it is arranged in the gas supplying means 108. In embodiments, both the means for increasing the pressure 108, e.g. the tube, and the sluice vessel 104 are equipped with means for measuring the pressure.

The supply of gas (or pressure of the supplied gas) into the compartment 107 may be controlled and adjusted in response to the measured pressure, for example by means of the valve 111.

In embodiments, the second discharge valve 106 is configured to be opened in one step or in multiple steps.

In cases where the second discharge valve 106 is configured to be opened in one step, the discharge of biomass from the compartment 107 is performed simultaneously with the drop in pressure from the compartment to lower pressure, typically atmospheric pressure. In cases where the second discharge valve 107 is opened in multiple steps, the biomass is gradually and more gently discharged from the sluice vessel.

The second discharge valve 106 may be configured to be opened in multiple steps, such as two steps, wherein the first step is conducted at a lower speed than the following step(s).

This way, the time taken for the pressure to decrease from a higher compartment pressure to a lower pressure, e.g. atmospheric pressure is prolonged. The discharge is thereby performed in a more controlled manner.

The reactor vessel 101 in figure 1 is a vertical reactor vessel. However, the pretreatment arrangement of the present disclosure is not limited to the use of a vertical reactor vessel. Horizontal reactor vessels are also conceivable for the purpose of the present disclosure.

As illustrated in figure 1, the reactor vessel 101 is a vertical reactor vessel extending along a longitudinal center line 118. The biomass 119 fed into the reactor vessel 101 flows from the inlet 102 to the outlet 103 by means of gravity, and does not require additional feeding or mixing means to support the flow in the reactor vessel 101.

Typically, the reactor vessel 101 is cylindrical and has a circular or oval cross-section, which cross-section area may be constant or vary along the longitudinal center line.

In embodiments, the reactor vessel 101 has a rotational symmetry with respect to the longitudinal center line 118.

Preferably, the sluice vessel 104 is adapted for steam explosion. In other words, the reactor vessel 101 is a unit in which the biomass is pretreated, and the sluice vessel 104 is a unit in which steam explosion of the pretreated biomass is carried out. The reactor vessel 101 may be adapted to operate under process conditions optimal for the pretreatment, whereas the more harsh or severe pretreatment conditions (large pressure increases and pressure releases) are performed in the sluice vessel 104. This way, the risk of deposit formation and formation of inhibitory agents are prevented within the reactor vessel. A more controlled steam explosion is thereby achieved.

In embodiments, the sluice vessel 104 is adapted to withstand pressures up to 100 bar, e.g. up to 75 bar, e.g. up to 50 bar.

The pretreatment arrangement 100 may comprise more than one sluice vessel. In embodiments, the sluice vessel 104 is a first sluice vessel and the pretreatment arrangement further comprises at least a second sluice vessel; the second sluice vessel being arranged in parallel with the sluice vessel 104 or downstream of the first sluice vessel 104 (not shown).

In case the second sluice vessel is arranged in parallel with the first sluice vessel 104, both the first and the second sluice vessel are arranged in fluid communication with the reactor vessel 101. If the second sluice vessel is arranged in series with the first sluice vessel 104, the second sluice vessel is arranged downstream of and in fluid communication with the first sluice vessel 104. In such embodiments, the biomass may be subjected to a step-wise pressure increase and decrease. For example, the pressure of the first compartment 107 of the first sluice vessel 104 may be lower than the pressure of the compartment of the second sluice vessel, and vice versa. A first steam explosion step may occur upon discharge from the first sluice vessel 104 and a second steam explosion step may occur upon discharge from the second sluice vessel.

As illustrated in figure 1, the arrangement 100 for pretreatment of lignocellulosic biomass may further comprise a scraping device 111. The scraping device 111 secures a continuous flow of biomass in the reactor vessel, while scraping deposits formed on the interior walls 112 of the reactor vessel 101. The scraping device 111 prevents build-up of deposit inside the reactor vessel 101, and the full interior volume of the reactor vessel 101 can therefore be utilized for the pretreatment of lignocellulosic biomass.

The scraping device 111 may comprise a shaft 113 and at least two scraping blades 114 extending from the shaft 113. The scraping blades 114 are preferably configured to follow the contour of, without contacting the interior walls 112 of at least a portion of the lower portion 115 of the reactor vessel 101. The shaft 113 may be arranged outside of the reactor vessel 101 or may be configured to extend into the upper portion 117 of the reactor vessel 101. Preferably, the shaft 113 does not extend into the lower portion 115 of the reactor vessel 101. In other words, the shaft 113 does not extend into a portion of the vessel where slurry is present. The reason is that a shaft 113 extending into the slurry may form an additional surface onto which deposit may form and develop.

The scraping blades 114 are arranged to rotate about the longitudinal center line 118 and are preferably arranged to provide an efficient scraping of the interior reactor walls 112 without risking that these become damaged by the blades. Therefore, a small gap should preferably be provided between scraping blades 114 and the interior walls 112.

In embodiments, the scraping blades 114 are arranged at a distance, d1, from the interior walls 112 of the reactor vessel 101, wherein the distance, d1, corresponds to from 0.5 to 20 %, preferably from 2 to 15 % of the inner diameter of the reactor vessel 101.

In figure 1, the exterior walls of the reactor vessel 101 are denoted 116. The exterior walls 116 may be configured to taper towards the outlet 103 in the lower portion 115 of the reactor vessel. This way, an improved discharge of biomass from the outlet 103 towards the sluice vessel 104 is achieved.

The arrangement 100 for pretreatment of lignocellulosic biomass may further comprise a gas valve 120 configured to remove gas from the reactor vessel 101.

The gas valve 120 preferably has an adjustable opening configuration. The gas valve 120 may be attached to the reactor vessel 101 or connected to the reactor vessel 101 by means of a tube (the latter of which is illustrated in figure 1).

The reactor vessel 101 may further comprise measuring means 121 for measuring a number of process parameters of the pretreatment in the reactor vessel 101. Such process parameters include at least a temperature parameter and a pressure parameter.

The pretreatment arrangement 100 may further comprise gas flow control means 122 configured to adjust the outflow of gas from the gas valve 120 in response to the measured process parameters. This way, a controlled outflow of gas from the reactor vessel 101 is achieved. Accordingly, a more controlled pretreatment is achieved. The temperature and pressure have been identified as key parameters, together sufficient for achieving stable pretreatment conditions, which implies an efficient pretreatment and reduced formation of deposits on the interior walls of the reactor.

The relationship between the pressure and the temperature is preferably monitored throughout the pretreatment reaction, and when the temperature (or the pressure) deviates from a desired, preferably substantially constant, pressure-to-temperature relationship, this is typically an indication that gases, e.g. inert gases, have started to accumulate within the reactor vessel 101. Such gases may then be removed from the reactor vessel 101 by means of the gas valve 120. The outflow of gas from the gas valve 120 may be adjusted and regulated in response to deviations in temperature or pressure.

In embodiments, the gas flow control means 122 is configured to adjust the outflow of gas from the gas valve 120 in response to the relationship between the temperature and pressure, e.g. expressed as a ratio between temperature and pressure, so as to achieve a controlled flow of gas out from the reactor vessel.

Furthermore, the gas flow control means 122 may be configured to determine a ratio between the temperature parameter and the pressure parameter and to adjust the outflow of gas from the gas valve 120 in response to the determined ratio. The gas flow control means 122 may be configured to adjust the outflow of gas from the gas valve 120 if the determined ratio deviates from a predetermined reference ratio interval for the pretreatment.

By adjusting the outflow of gas from the gas valve 120 in response to the relationship between the temperature and pressure, the temperature and pressure, or the ratio between temperature and pressure, can be held within a predetermined interval of deviation (basically constant, if the interval is comparatively narrow) for the specific pretreatment to be carried out.

Such pretreatment arrangements will counteract or compensate for imbalance between the temperature and pressure within the reactor caused by the liberation of gases from the biomass during degradation or partial degradation, and which is particularly problematic if the pretreatment is carried out by applying steam or additional catalysts, particularly gaseous catalysts, leading to an excess amount of accumulated gases in the reactor.

In embodiments, the pretreatment arrangement 100 comprises a flow meter 123 configured to measure the outflow of gas from the reactor vessel 101. The flow meter 123 may indicate that the flow of gas is too high or too low, and the gas flow control means 122 may be configured to adjust the opening of the gas valve 120 in response to the measured outflow of gas.

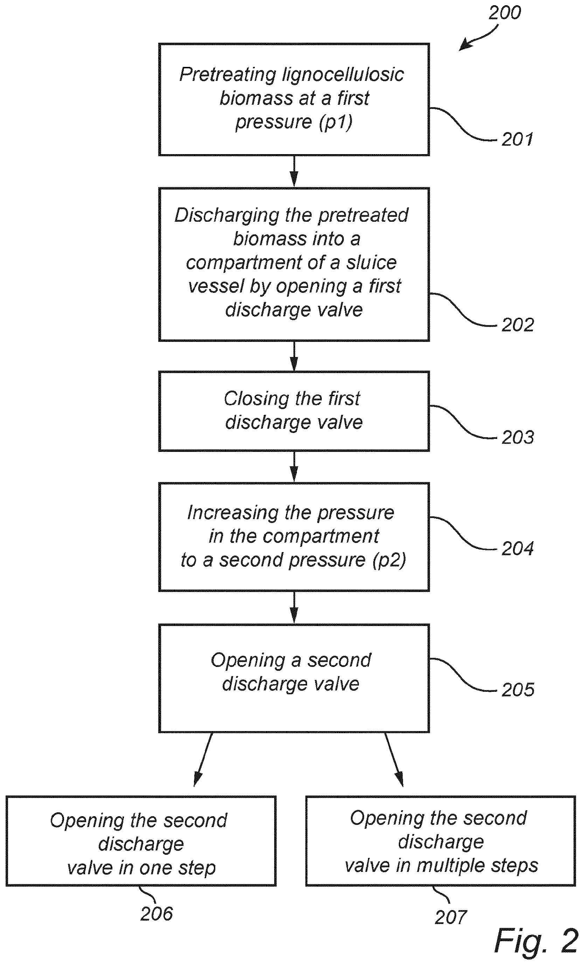

According to another aspect, the present disclosure further provides a method for pretreatment of lignocellulosic biomass. The steps of the method are schematically outlined in figure 2. The references related to the pretreatment arrangement of figure 1 are kept throughout the description of the method.

The method 200 for pretreatment of lignocellulosic biomass comprises
a) pretreating the lignocellulosic biomass in a pretreatment arrangement 100 at a first pressure (p₁), wherein the pretreatment arrangement 100 comprises a reactor vessel 101 having an upstream inlet 102 for receiving biomass and a downstream outlet 103 for discharging biomass; the pretreatment arrangement further comprising a sluice vessel 104 comprising a first discharge valve 105, a second discharge valve 106 arranged downstream of the first discharge valve 105, and a compartment 107 arranged between the first 105 and the second 106 discharge valve (this step is illustrated by 201 in figure 2),
b) discharging the biomass into the compartment 107 by opening the first discharge valve 105 (illustrated by 202 in figure 2),
c) closing the first discharge valve 105 (illustrated by 203 in figure 2),
d) increasing the pressure in the compartment 107 to a second pressure (p₂) (illustrated by 204 in figure 2),
e) discharging the biomass by opening the second discharge valve 106 (illustrated by 205 in figure 2).

The pressure of step d) may be increased by supplying gas, such as steam, to the compartment 107. The gas may be supplied by means of a tube.

The second pressure (p2) may be 1-40 bar, such as 2-30 bar, such as 4-20 bar higher than the first pressure (p1). The pressure (p1) may be 15-30 bar, such as 10-20 bar. The gas for increasing the pressure inside the sluice vessel may be steam, such as pressurized steam. The higher the pressure of the saturated steam, the higher is also the temperature. The pressure of the saturated steam may be 30-55 bar, such as 35-45 bar. A second pressure (p2) that is higher than p1 allows for an efficient division of the lignocellulosic biomass into small particles upon release of pressure to lower pressure, typically atmospheric pressure.

The residence time, t2, of the lignocellulosic biomass in the compartment 107 in step d) may be from 1 second to 10 minutes, e.g. from 5 seconds to 5 minutes, e.g. from 5 to 60 seconds.

The residence time, t1, in the reactor vessel 101 is preferably 2-500 times longer, such as 50-500 times longer, such as 100-400 times longer, such as 200-400 times longer, such as 300-400 times longer than the residence time, t2, in the sluice vessel 104 (step d). The residence time in the reactor vessel is suitably 3-60 minutes, such as 5-50 minutes, such as 10-40 minutes. The residence time, t2, in the sluice vessel is preferably limited so that burning of sugars and decomposition of sugars and lignin into undesired compounds is avoided.

Pressures above 20-22.5 bar, i.e. temperatures above 215-220 °C, in combination with standard residence times e.g. 30 minutes, inside the reactor vessel may be harmful for the lignocellulosic biomass and may cause burning of sugars and decomposition of sugars and lignin into undesired compounds.

The pressure may be monitored to ensure that the desired pressure is obtained in the sluice vessel. This may be achieved by the provision of means of measuring pressure in the sluice vessel 104.

As illustrated by the step 206 in figure 2, the second discharge vessel 105 may be opened in one step, allowing the pressure to drop to a lower pressure (usually atmospheric pressure) than the pressure, p2, of the sluice vessel 104 at the same time as the lignocellulosic biomass is discharged.

Alternatively, the second discharge vessel 106 is opened in multiple steps (see 207 in figure 2) so that the pressure is dropped to lower pressure, typically atmospheric pressure while the lignocellulosic biomass is gradually discharged. For example, the first step may be conducted at a lower speed than the following steps, allowing for a milder pretreatment as the decrease in pressure is allowed to take more time.

By increasing the pressure inside the sluice vessel 104 and thereafter decreasing the pressure upon opening of the second discharge vessel 106, a steam explosion is conducted when the lignocellulosic biomass exits the sluice vessel 104.

In the context of the present disclosure steam explosion refers to an increase of pressure in one or several step(s) followed by a rapid decrease of pressure that causes the lignocellulosic biomass to explode into smaller pieces.

As an illustrative example, lignocellulosic biomass may be steam heated at a certain temperature and pressure for a given time, e.g. 205°C, at a pressure of 18 bar for 10 minutes, followed by a rapid pressure increase, e.g. to twice the amount of pressure for a short period of time, e.g. during less than 60 seconds, followed by discharge to a lower pressure, e.g. atmospheric pressure, causing the lignocellulosic biomass to explode. In figure 1a, the addition of steam into the reactor vessel 101 is denoted 125.

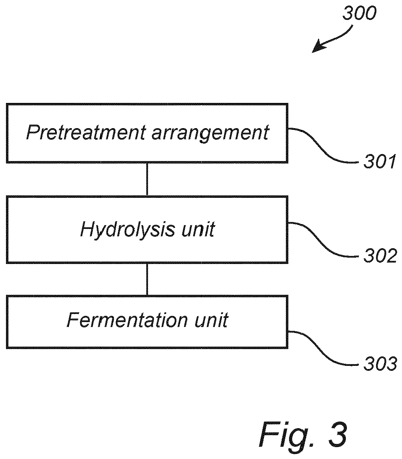

With reference to figure 3, the present disclosure further provides a system 300 for treatment of lignocellulosic biomass comprising a pretreatment arrangement 301 for pretreatment of lignocellulosic biomass according to the first aspect of the present disclosure, a hydrolysis unit 302 arranged downstream of and in fluid communication with the pretreatment arrangement 301, and optionally, a fermentation unit 303, such as a fermentation vessel, arranged downstream of and in fluid communication with the hydrolysis unit 302. The system 300 may comprise additional units and components known to those skilled in the art. For example, a separation unit may be arranged after pretreatment, such as between the pretreatment arrangement 301 and the hydrolysis unit 302, and/or between the hydrolysis unit 302 and the fermentation unit 303.

In the hydrolysis unit, the pretreated biomass is subject to enzymatic hydrolysis by means of saccharification enzymes. Fermentation of the hydrolysate into a target chemical is typically performed by means of fermenting organism, such as bacteria and/or yeast. The system 300 may also comprise a product recovery unit, such as distillation or ion exchange chromatography, arranged downstream of and in fluid communication with the fermentation unit 303.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A pretreatment arrangement (100) for pretreatment of lignocellulosic biomass comprising:
a) a reactor vessel (101) having an upstream inlet (102) for receiving biomass and a downstream outlet (103) for discharging biomass,
b) at least one sluice vessel (104) arranged downstream of and in fluid communication with said outlet (103), wherein said sluice vessel (104) comprises a first discharge valve (105), a second discharge valve (106) arranged downstream of said first discharge valve (105) and a compartment (107) arranged between said first (105) and said second (106) discharge valve; said first (105) and said second (106) discharge valves being configured to be operable between an open and a closed position, and
c) means (108) for increasing the pressure in said compartment (107) of said sluice vessel (104).

2. The pretreatment arrangement (100) according to claim 1, wherein said sluice vessel (104) is attached to said reactor vessel (101) or is connected to said reactor vessel by means of a pipe (109).

3. The pretreatment arrangement (100) according to claim 1 or claim 2, wherein said compartment (107) comprises a tank (107b) and/or a pipe (110).

4. The pretreatment arrangement (100) according to any one of the preceding claims, wherein said means (108) for increasing the pressure is a means for supplying gas, such as steam, to said compartment (107).

5. The pretreatment arrangement (100) according to any one of the preceding claims, wherein said sluice vessel (104) comprises means for measuring the pressure in said sluice vessel (104).

6. The pretreatment arrangement (100) according to any one of the preceding claims, wherein said second discharge valve (106) is configured to be opened in one step or in multiple steps.

7. The pretreatment arrangement (100) according to claim 6, wherein said second discharge valve (106) is configured to be opened in multiple steps, such as two steps, wherein the first step is conducted at a lower speed than the following step(s).

8. The pretreatment arrangement (100) according to any one of the preceding claims, wherein said reactor vessel (101) is a vertical reactor vessel.

9. The pretreatment arrangement (100) according to any one of the preceding claims, wherein said sluice vessel (104) is adapted for steam explosion.

10. The pretreatment arrangement (100) according to any one of the preceding claims, wherein said sluice vessel (104) is a first sluice vessel and wherein said pretreatment arrangement (100) further comprises at least a second sluice vessel; said second sluice vessel being arranged in parallel with said first sluice vessel (104) or downstream of said first sluice vessel (104).

11. The pretreatment arrangement (100) according to any one of the preceding claims, wherein said reactor vessel (101) further comprises a scraping device (111) configured to scrape deposits formed on the interior walls (112) of said reactor vessel (101).

12. The pretreatment arrangement (100) according to any one of the preceding claims, further comprising a gas valve (120) configured to remove gas from said reactor vessel (101).

13. A method for pretreatment of lignocellulosic biomass comprising:
a) pretreating said lignocellulosic biomass in a pretreatment arrangement (100) at a first pressure (p₁), wherein said pretreatment arrangement (100) comprises a reactor vessel (101) having an upstream inlet (102) for receiving biomass and a downstream outlet (103) for discharging biomass; said pretreatment arrangement (100) further comprising a sluice vessel (104) comprising a first discharge valve (105), a second discharge valve (106) arranged downstream of said first discharge valve (105), and a compartment (107) arranged between said first (105) and said second (106) discharge valve,
b) discharging said biomass into said compartment (107) by opening said first discharge valve (105),
c) closing said first discharge valve (105),
d) increasing the pressure in said compartment (107) to a second pressure (p₂),
e) discharging said biomass by opening said second discharge valve (106).

14. The method according to claim 13, wherein the pressure is increased by supplying gas, such as steam to said compartment (107).

15. The method according to claim 13 or claim 14, wherein said second pressure, p₂, is 1-40 bar, such as 2-30 bar, such as 4-20 bar higher than said first pressure, p₁.

16. A system (300) for treatment of lignocellulosic biomass comprising a pretreatment arrangement (301) according to any one of claims 1-12 and a hydrolysis unit (302) arranged in fluid communication with and downstream of said pretreatment arrangement (301), and optionally, a fermentation unit (303) arranged in fluid communication with and downstream of said hydrolysis unit (302).
